# EUROPEAN PATENT SPECIFICATION

(11) **EP 4 048 349 B1**
(45) Date of publication and mention of the grant of the patent: **09.07.2025**
(21) Application number: 20789990.7
(22) Date of filing: 14.10.2020
(51) Int. Cl.: A61M 5/315, A61M 5/20, A61M 5/32

(54) **SAFETY INJECTION DEVICE**
SICHERHEITSINJEKTIONSVORRICHTUNG
DISPOSITIF D'INJECTION DE SÉCURITÉ

(30) Priority: 23.10.2019 EP 19315130
(43) Date of publication of application: 31.08.2022
(73) Proprietor: Becton Dickinson France, 38800 Le Pont de Claix (FR)
(72) Inventor: ZELLER, Benoît, 74940 Annecy-le-Vieux (FR); BERNEDE, Gilles, 74930 Arbusigny (FR)
(74) Representative: Roux, Stéphane
(86) International application number: PCT/EP2020/078928
(87) International publication number: WO 2021/078610

(56) References cited:
- WO-A1-2015/121081
- WO-A1-2016/128977
- US-A1- 2003 212 380
- US-A1- 2016 106 920

## Description

The present invention relates to a medical injection system for self-administration of a substance or a drug composition.

### Background Art

Medical injection systems allowing self-administration of a drug composition have been developed so that patients having non-curable or long-term diseases can administer their own drug composition without the need of any medical staff. Consequently, these medical injection systems usually provide a simplified operation, for example by combining several steps into a single distal movement.

Such a medical injection system is usually provided in a position in which the injection needle intended to deliver the drug composition is hidden or covered by the medical injection system and can perform at least the following steps:
- a first step of needle pricking, i.e. inserting the injection needle into the patient's body,
- a second step of injection, i.e. injecting or delivering the drug composition through the injection needle into the patient's body.

However, a problem may occur in that the injection step is triggered before the end of the needle pricking step so that the injection needle may not be inserted into the patient's body or may be inserted at an inappropriate depth of the patient's skin. Such a failed administration often results in wasting valuable drug composition and is inconvenient for the patient.

The purpose of the present invention is to solve the above-mentioned problem in order to propose a medical injection system of a low cost and simple operation, and able to minimize the number of failed administrations. Relevant prior art can be found under WO 2015/121081, US 2016/106920, US 2003/212380, and WO 2016/128977.

### Presentation of the Invention

To this end, a first aspect of the invention is an injection device for injecting a substance into a body, comprising:
(a) a barrel adapted to receive the substance and provided with a stopper in sliding engagement inside the barrel and with an injection needle;
(b) a plunger rod configured to push the stopper and moveable in order to inject the substance when a pushing force is applied to the plunger rod,
(c) a locking clip fixed relative to the barrel and provided with at least two jaws adapted for engaging the plunger rod in a primary position of the plunger rod, so as to prevent an injection movement of the plunger rod with regard to the barrel, and
(d) a needle shield comprising a triggering portion, said needle shield being configured in sliding engagement with respect to the injection needle, so as to define an initial position in which the needle shield covers the injection needle and a retracted position in which the needle shield does not cover at least a portion of the injection needle;
wherein the triggering portion is configured to open the locking clip when the needle shield moves from the initial position to the retracted position, so as to disengage the locking clip from the plunger rod and to allow the injection movement of the plunger rod.

Due to the engagement of the locking clip with the plunger rod, at least the injection movement and preferably any movement of the plunger rod is prevented as long as the needle shield is not in the retracted position and the injection needle is not inserted into the body at a predetermined depth. The direct engagement of the needle shield with the locking clip preferably occurs in an end portion of the movement of the needle shield, for example, from 80, 85 or 90% of the movement of the needle shield toward the retracted position. For example, the two jaws can be rotated or deflected out of a longitudinal axis of the injection device or of a longitudinal shaft of the plunger rod. More than two jaws may be provided, such as three or four jaws.

Advantageously, the plunger rod comprises an intermediate stop and the two jaws of the locking clip are arranged to engage said intermediate stop in the primary position of the plunger rod. The intermediate stop may comprise one or several abutment surfaces, grooves or protrusions extending from the longitudinal shaft of the plunger rod. The jaws may have a thick edge in order to increase resistance to a distal force when the locking clip is not open.

Advantageously, the needle shield is further moveable to a safety position in which the needle shield covers permanently the injection needle, and
the injection device further comprises :
   (e) an operating stop configured to prevent the needle shield to move to the safety position,
   (f) a flexible support supporting the operating stop and fixed with regard to the barrel,
   (g) a deflecting member configured to contact the operating stop and/or the flexible support and to receive the pushing force from the plunger rod when the plunger rod is moved to a threshold position;
wherein the deflecting member is further configured to move under said pushing force so as to deflect the flexible support and to release the operating stop.

Such a safety position of the needle shield may be located distally from the initial position of the needle shield. It thus allows to prevent or limit accidental needle pricking and to permit a safe disposal after use of the injection device. In addition, since the safety position of the needle shield is only adopted after the threshold position of the plunger rod has been met, the user can change the injection site, i.e. the place in the body in which the substance is injected, at any time before this threshold position of the plunger rod. The threshold position can be located at 70 to 95% of the injection movement of the plunger rod, preferably, 80 to 90%.

Advantageously, the deflecting member comprises:
an abutment surface configured to receive the pushing force of the plunger rod when the plunger rod is in the threshold position and
a sloped portion configured to slide on the flexible support and/or the deflecting member so as to deflect the flexible support when the deflecting member moves under the pushing force of the plunger rod.

Such a deflecting member is easy to produce and to assemble. It also allows a reliable operation.

Advantageously, the plunger rod comprises a pushing surface adapted to transmit the pushing force to the abutment surface of the deflecting member. The plunger rod is preferably adapted to be pushed by a user, for example thanks to a thumb surface. Such a plunger rod is convenient and reliable during operation and simple to be produced.

Advantageously, the injection device further comprises a case accommodating the locking clip, the barrel, the needle shield and the deflecting member, and the operating stop and the flexible support are fixed to or are part of the case. Preferably, the barrel is held and fixed inside the case, for example thanks to rigid clips clipping a flange or another portion of the barrel.

Advantageously, the case comprises a distal unit accommodating the needle shield and the barrel and a proximal unit accommodating the locking clip and the deflecting member. Such a two-part case is easy to manufacture and to assemble.

Advantageously, the distal unit of the case comprises a longitudinal window giving a visual access to the barrel. Since the deflecting member and the locking clip are accommodated into the proximal portion of the case, a direct view can be obtained to check the substance before injection and to follow the stopper movement during injection.

Advantageously, elastic means are arranged in pushing engagement with the needle shield so as to move the needle shield from the retracted position to the initial position and/or to the safety position. Preferably, the elastic means are accommodated in the proximal unit of the case which allows not to mask the view through the window. These elastic means may be a cylindrical spring arranged around the deflecting member and the locking clip, which allows to build a simple and thin injection device that can be easily transported and stored.

Advantageously, the deflecting member comprises a longitudinal tab or another form of tab adapted to pop up from the case when the deflecting member moves under the pushing force of the plunger rod. This tab thus acts as an end-of-dose indicator in order to provide a clear signal to the user that the injection has been completed.

Advantageously, the injection device comprises safety locking means adapted to lock the needle shield to the case when the needle shield is in the safety position. For example, the safety locking means comprise at least one peg provided on one of the case and the needle shield and at least one recess provided on the other of the case and the needle shield, the recess being adapted to accommodate and hold the peg in the safety position of the needle shield.

Advantageously, the needle shield further comprises at least one longitudinal arm extending along the barrel and comprising an extremity provided with the triggering portion. Such a needle shield is easy and inexpensive to manufacture and allows to provide the longitudinal window on the case.

Other features and advantages of the present invention will appear more clearly from the following detailed description of particular nonlimiting examples of the invention, illustrated by the appended drawings in which:
- figure 1 represents a perspective view of an injection device according to an example of the present invention, in a ready-to-use state;
- figure 2 represents a perspective view of the injection device of Fig. 1 without a proximal unit of the case;
- figure 3 represents a cross section view of the injection device of Fig. 1;
- figure 4 represents a perspective view of a plunger rod of the injection device of Fig. 1;
- figure 5 represents a perspective view of a distal unit of the case of the injection device of Fig. 1;
- figure 6 represents a perspective view of a needle shield of the injection device of Fig. 1.
- figures 7A-7B represent two perspective views of a locking clip of the injection device of Fig. 1,
- figure 8 represents a perspective view of a deflecting member of the injection device of Fig. 1,
- figure 9 represents a partial side view of the injection device of Fig. 2 in a primary position, without the cylindrical spring,
- figure 10 represents a side view of the injection device of Fig. 2 during pricking,
- figure 11 represents a partial side view of the injection device of Fig. 2 during pricking, illustrating the opening of the locking clip,
- figure 12 represents a partial side view of the injection device of Fig. 2 during injection,
- figure 13 represents a partial side view of the injection device of Fig. 2, in a threshold position of the plunger rod, without the locking clip,
- figure 14 represents a side view of the injection device of Fig. 2 in a safety position of the needle shield.

### Detailed Description of the Invention

The present injection system is intended for administration of a substance such as one or several parenteral drug compositions by a user such as a medical caregiver or preferably by a patient with a simplified operation.

As such, in this application, the distal direction must be understood as the direction of injection with reference to the medical injection device, and the proximal direction is the opposite direction, i.e. the direction toward the hand of the user, i.e. the medical caregiver or the patient. In addition, drug compositions must be understood as all kinds of injectable drug composition adapted for therapeutics, aesthetics, preventive or diagnosis applications.

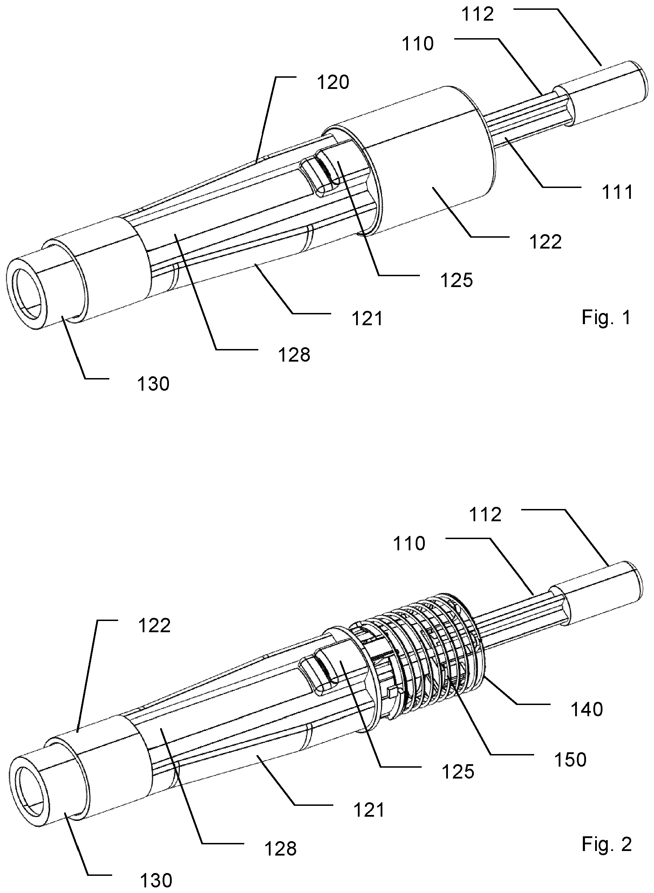

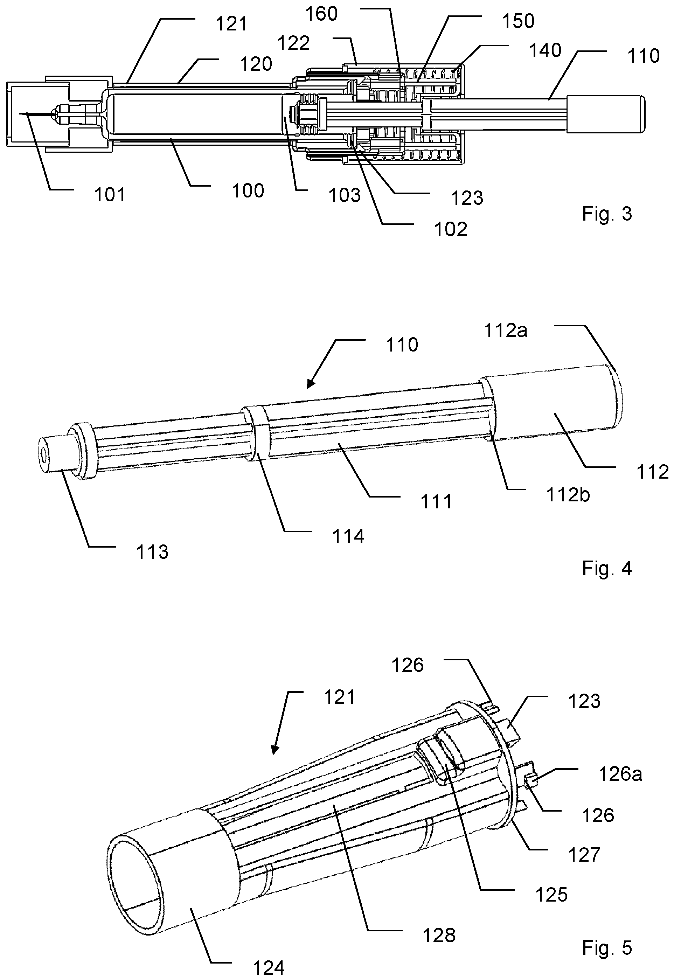

With reference to Figs. 1-3 is shown an injection device according to a preferable example of the present invention. The injection device comprises a barrel 100 (visible in Fig. 3) of a syringe, provided with an injection needle 101, a distal flange 102 and a stopper 103 in sliding engagement inside the barrel. The injection needle 101 may be glued, stacked or removably fixed to the barrel 100 but is preferably glued or stacked. The barrel may be in rigid plastic, metal but preferably in glass.

A plunger rod 110 is coupled to the stopper 103 or at least configured to push the stopper 103 and is preferably adapted to be moved manually, for example by the user. As it is known, a distal force applied on the plunger rod 110 results in moving the stopper 103 in order to perform the injection of the substance. The plunger rod 110 is moveable in an injection movement from a primary, proximal position to a final, distal position. With reference to Fig. 4, the plunger rod 110 comprises a longitudinal shaft 111 having in the example of the figures a cross-shaped cross-section, a proximal portion 112, a distal coupling portion 113 and an intermediate stop 114.

The proximal portion 112 comprises at its extremity a thumb surface 112a adapted to be pushed by the thumb of a user and a pushing surface 112b. The distal coupling surface 113 is adapted to be coupled to the stopper 103, for example by clipping, gluing or screwing. The intermediate stop 114 and the pushing surface 112b are in the example of the figures transversal surfaces protruding from the cross-shaped longitudinal shaft 111.

With reference to Figs 1-3 and 5, the barrel 100 is accommodated and secured in a case 120 by its distal flange 102. The case comprises a distal unit 121 enclosing and holding the barrel 100 thanks to rigid clips 123 and a proximal unit 122 located around and proximally to the distal flange 102 of the barrel 100. The proximal unit 122 of the case 120 is omitted in Figs. 2 and 5. The distal unit 121 comprises a central cavity accommodating the barrel 100 and further comprises a distal ring 124 and side protrusions 125.

The side protrusions 125 are hollow and comprise each a through slot. One or several longitudinal windows 128 made of transparent material may be arranged on the case in order to give a visual access to the barrel 100. The distal unit 121 and the proximal unit 122 of the case 120 are clipped together but may also be screwed or glued. The distal unit 121 of the case 120 further comprises flexible supports 126 protruding from a proximal extremity 127 and provided with a proximal surface or protrusion acting as an operating stop 126a.

With reference to Figs. 1-3 and 6, a needle shield 130 is provided in sliding engagement within the case 120. The needle shield 130 comprises a safety tube 131 from which two rigid arms 132 protrude in the proximal direction. The rigid arms 132 each comprise an extremity provided with a stop portion 133 and a triggering portion 134 under the form of a triangular or sloped portion. In addition, the rigid arms 132 further comprise one and preferably two pegs 135 extending laterally from the rigid arms 132.

With regard to Figs. 2 and 3, the proximal unit 122 of the case 120 encloses elastic means under the form of a cylindrical spring 140 enclosing a locking clip 150 and a defecting member 160. The cylindrical spring 140 is adapted to be in pushing engagement on the proximal face of the stop portions 133 of the needle shield 130 and on an opposite surface of the proximal unit 122 of the case 120.

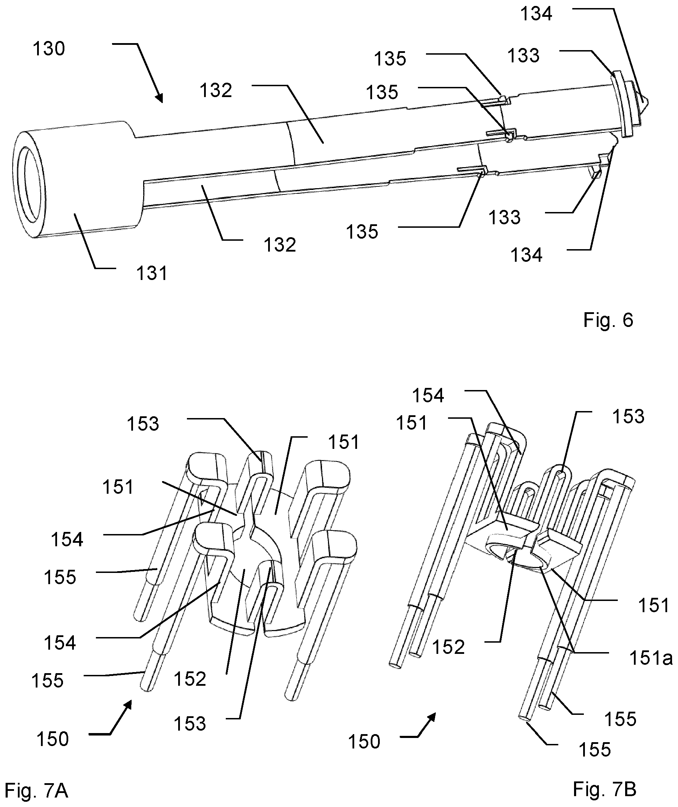

In Figs. 7A-7B, the locking clip 150 comprises two jaws 151 enclosing a central hole 152, the jaws being connected with each other by flexible intermediary portions 153 and each jaw is further connected thanks to flexible connecting portions 154 to two legs 155 extending distally. The legs of the locking clip 150 are intended to contact or to be fixed to the proximal extremity 127 of the distal unit 121 of the case 120. The jaws 151 can have a thick edge 151a around the central hole 152.

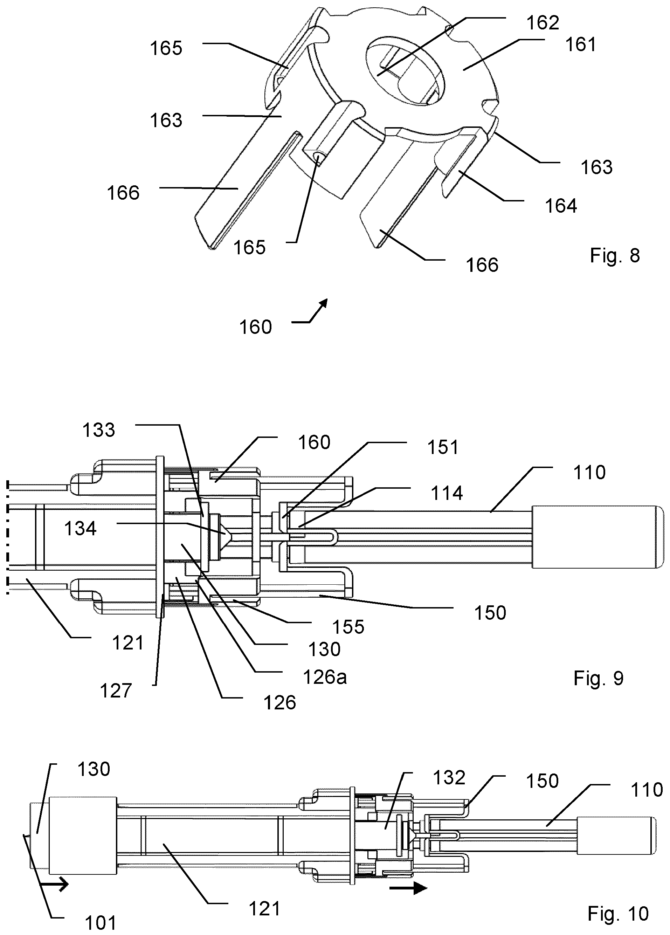

With reference to Fig. 8, the deflecting member 160 comprises an abutment surface 161 having a through hole 162 and two side surfaces 163. The side surfaces 163 further comprise sloped portions 164, wherein the slope or the thickness increases from the distal side to the proximal side of the sloped portion, i.e. from bottom to top in Fig. 8. The side surfaces 163 have each two through grooves 165 intended to accommodate the legs 155 of the locking clip 150 and a longitudinal tab 166.

With regard to Fig. 9, a proximal area of the injection device is shown without the cylindrical spring 140 and the proximal unit 122 of the case 120, which are omitted. The locking clip 150 is visible with the legs 155 extending distally through the through grooves 165 of the locking clip 150 and contacting or fixed to the proximal extremity 127 of the distal unit 121 of the case 120. The deflecting member 160 contacts the flexible supports 126 and the operating stop 126a by its sloped portions 164 and its longitudinal tabs 166 are accommodated in the through slots of the side protrusions 125 of the case 120. The extremity of rigid arms 132 of the needle shield 130 is also extending further from the proximal extremity 127 of the case distal unit 121 and their stop portions 133 are in contact or abutment with the operating stops 126a.

In a ready-to-use state of the present injection device, illustrated in Figs. 1-3 and 9, the safety tube 131 of the needle shield 130 covers the injection needle 101 and a distal movement of the needle shield 130 is prevented by the contact between the stop portions 133 and the operating stop 126a. However, a proximal movement of the needle shield 130 is permitted. In addition, the plunger rod 110 is inserted through the locking clip 150 and the deflecting member 160 thanks to the central hole 152 and the through hole 162, respectively. The jaws 151 of the locking clip 150 are in contact with the intermediate stop 114. Consequently, a distal movement of the plunger rod 110 is prevented by the locking clip 150 and an injection cannot be performed in this ready-to-use state.

### Operation of the Injection Device

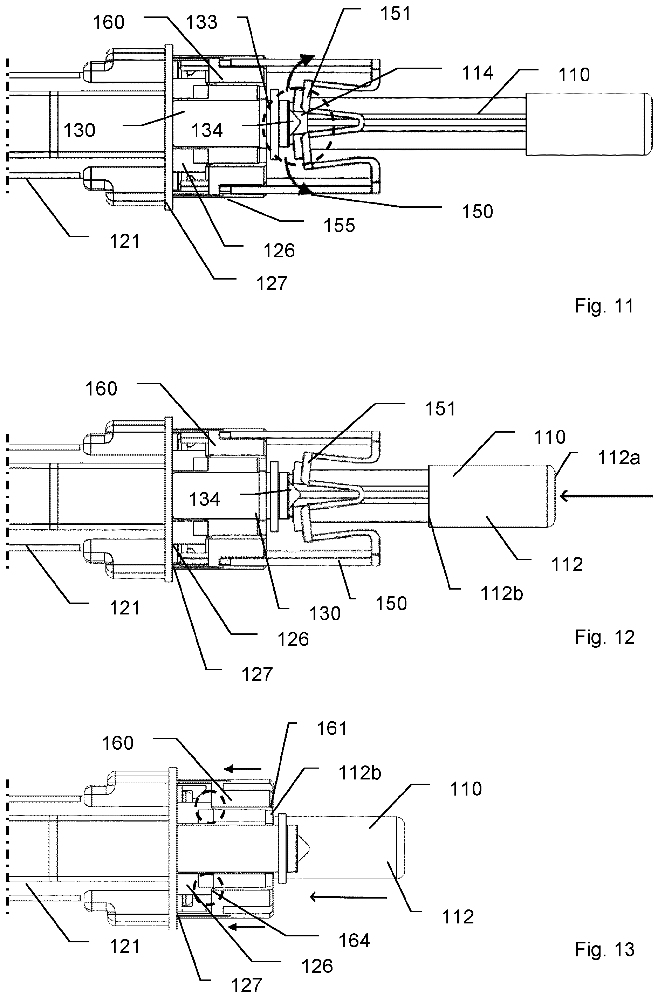

In operation, the injection device and in particular its distal extremity comprising the injection needle 101 is pressed on a body and the needle shield 130 moves proximally, thus allowing the injection needle 101 to penetrate or prick the body, as illustrated in Fig. 10. Consequently, the rigid arms 132 moves proximally according to the arrow of Fig. 10 and the triggering portions 134 comes in contact with the jaws 151 of the locking clip 150 (see Fig. 11).

Thanks to the proximal force applied on and between the jaws 151 by the triggering portion 134 of the needle shield 130 (see the circled area in Fig. 11), the jaws 151 start to rotate according to the arrows visible in Fig. 11. Consequently, the jaws 151 are progressively disengaged from the intermediate stop 114 of the plunger rod 110 by the proximal movement of the needle shield 130 resulting from the pricking of the body. At the end of the pricking i.e. at the end of the proximal movement of the needle shield 130 into the case 120, the jaws 151 are disengaged from the intermediate stop 114. Consequently, a distal force applied on the thumb surface 112a of the plunger rod 110 (see the arrow in Fig. 12) allows a distal movement of the plunger rod 110, thus performing the injection of the substance into the body by movement of the stopper 103 into the barrel 100.

At this moment or even during injection, the user can still change his mind regarding the injection site, in particular in case of pain or for convenience reasons. The injection device can be withdrawn from the body, the needle shield 130 moves back to its initial position covering the injection needle 101 and the pricking can be repeated on another injection site of the body.

In Fig. 13, most of the substance has been injected and the plunger rod 110 is in a threshold position, in which the pushing surface 112b of the proximal portion 112 of the plunger rod 110 is in contact with the abutment surface 161 of the deflecting member 160. The plunger rod 110 is not in its final, distal position and can still move distally. In addition, the sloped surface 164 of the deflecting member 160 are in contact with the flexible supports 126 and/or with the operating stop 126a of the case 120. Consequently, the continuous force applied on the plunger rod 110 by the user to perform the injection pushes the deflecting member 160 distally toward the distal unit 121 of the case 120 (see the arrows in Fig. 13) and the sloped surface 164 deflects the flexible supports 126 inwardly, i.e. toward the plunger rod longitudinal shaft 111 (see the circled areas of Fig. 13). Please note that the locking clip 150 is omitted in Fig. 13 for clarity reasons.

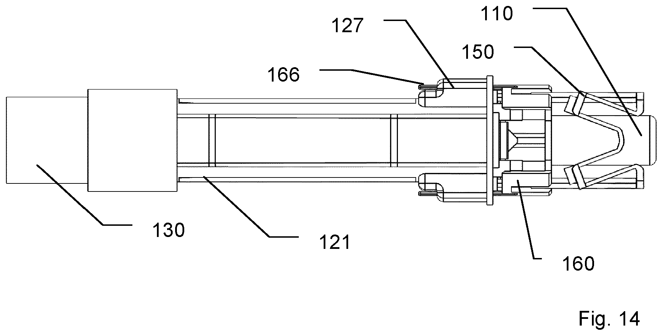

The deflection of the flexible supports 126 moves the operating stops 126a outside of their initial position and does not allow a contact with the stop portion 133 of the needle shield 130 anymore. Consequently, at the end of the injection, i.e. when the plunger rod 110 is in its final, distal position, the injection device can be withdrawn from the body and the needle shield 130 can move distally under the force of the cylindrical spring 140. Since no more contact can occur between the operating stops 126a and the stop portions 133, the needle shield 130 can move distally under the force of the cylindrical spring 140 until a contact occurs between the stop portion 133 and the proximal extremity 127 of the distal unit 121. The needle shield 130 is then in a safety position, located distally from the initial position and in which the safety tube 131 covers the injection needle 101, as visible in Fig 14.

In the safety position shown in Fig. 14, the needle shield 130 can be blocked or fixed with regard to the case 120 by engagement of the pegs 135 located on the rigid arms 132 of the needle shield 130, with corresponding recesses or abutments located on the inside surface of the case 120 (not represented), thus acting as safety locking means. In addition, thanks to the distal movement of the deflecting member 160 under the force transmitted by the plunger rod 110, the longitudinal tabs 166 protrude in the distal direction out of the side protrusion 125 of the case 120, thus acting as an end-of-dose indicator.

It is, of course, understood that obvious improvements and/or modifications for one skilled in the art may be implemented, still being under the scope of the invention as it is defined by the appended claims.

Even if the illustrated geometries of the different parts of the present injection device are preferable as being efficient and easy to produce, such parts are not limited to these specific designs. In particular, the deflecting member can have any specific design provided its function of deflecting the flexible supports can be performed. The locking clip can also adopt a variety of designs with one or several jaws.

In addition, the elastic means are not limited to one or two cylindrical springs but may also comprise any form of spring or elastic means. The external surface of the case may have an ergonomic relief and may comprise over-moulded surfaces in smooth material.

The materials intended to build such an injection device are polypropylene, polycarbonate and/or acrylonitrile-butadiene-styrene. The barrel may comprise glass or plastic. Colour may be provided on various parts of the injection device, in particular on the tab acting as the end-of-dose indicator. The window may be provided with graduations or indicators.

## Claims

1. An injection device for injecting a substance into a body, comprising:
a barrel (100) adapted to receive the substance and provided with a stopper (103) in sliding engagement inside the barrel (100) and with an injection needle (101);
a plunger rod (110) configured to push the stopper (103) and moveable in order to inject the substance when a pushing force is applied to the plunger rod (110),
a locking clip (150) fixed relative to the barrel (100) and provided with at least two jaws (151) adapted for engaging the plunger rod (110) in a primary position of the plunger rod (110), so as to prevent an injection movement of the plunger rod (110) with regard to the barrel (100), and
a needle shield (130) comprising a triggering portion (134), said needle shield (130) being configured in sliding engagement with respect to the injection needle (101), so as to define an initial position in which the needle shield (130) covers the injection needle (101) and a retracted position in which the needle shield (130) does not cover at least a portion of the injection needle (101);
wherein the triggering portion (134) is configured to engage and open the locking clip (150) when the needle shield (130) moves from the initial position to the retracted position, so as to disengage the locking clip (150) from the plunger rod (110) and to allow the injection movement of the plunger rod (110).

2. The injection device according to the previous claim,
wherein the plunger rod (110) comprises an intermediate stop (114) and the two jaws (151) of the locking clip (150) are arranged to engage said intermediate stop (114) in the primary position of the plunger rod (110).

3. The injection device according to any of the previous claims, wherein
the needle shield (130) is further moveable to a safety position in which the needle shield (130) covers permanently the injection needle (101), and
the injection device further comprises :
an operating stop (126a) configured to prevent the needle shield (130) to move to the safety position,
a flexible support (126) supporting the operating stop (126a) and fixed with regard to the barrel (100),
a deflecting member (160) configured to contact the operating stop (126a) and/or the flexible support (126) and to receive the pushing force from the plunger rod (110) when the plunger rod (110) is moved to a threshold position;
wherein the deflecting member (160) is further configured to move under said pushing force so as to deflect the flexible support (126) and to release the operating stop (126a).

4. The injection device according to the previous claim, wherein the deflecting member (160) comprises:
an abutment surface (161) configured to receive the pushing force of the plunger rod (110) when the plunger rod is in the threshold position (110) and
a sloped portion (164) configured to slide on the flexible support (126) and/or the operating stop (126a) so as to deflect the flexible support (126) when the deflecting member (160) moves under the pushing force of the plunger rod (110).

5. The injection device according to the previous claim, wherein the plunger rod (110) comprises a pushing surface (112b) adapted to transmit the pushing force to the abutment surface (161) of the deflecting member (160).

6. The injection device according to any one of claims 3 to 5, wherein the injection device further comprises a case (120) accommodating the locking clip (150), the barrel (100), the needle shield (130) and the deflecting member (160), and wherein the operating stop (126a) and the flexible support (126) are fixed to or are part of the case (120).

7. The injection device according to the previous claim, wherein the case (120) comprises:
a distal unit (121) accommodating the needle shield (130) and the barrel (100) and a proximal unit (122) accommodating the locking clip (150) and the deflecting member (160).

8. The injection device according to the previous claim, wherein the distal unit (121) of the case (120) comprises a longitudinal window (128) giving a visual access to the barrel (100).

9. The injection device according to any one of the previous claims, wherein the injection device further comprises elastic means arranged in pushing engagement with the needle shield (130) so as to move the needle shield (130) from the retracted position to the initial position and/or to the safety position.

10. The injection device according to the previous claim when depending on claims 7 to 9, wherein the elastic means are accommodated in the proximal unit (122) of the case (120).

11. The injection device according to claim 6 or any one of claims 7 to 10 when they depend on claim 6, wherein the deflecting member (160) comprises a longitudinal tab (166) adapted to pop up from the case (120) when the deflecting member (160) moves under the pushing force of the plunger rod (110).

12. The injection device according to any one of claims 6 to 11, further comprising safety locking means adapted to lock the needle shield (130) to the case (120) when the needle shield (130) in the safety position.

13. The injection device according to the previous claim, wherein the safety locking means comprise at least one peg (135) provided on one of the case (120) and the needle shield (130) and at least one recess provided on the other of the case (120) and the needle shield (130), the recess being adapted to accommodate and hold the peg in the safety position of the needle shield (130).

14. The injection device according to any one of the previous claims, wherein the needle shield (130) further comprises at least one longitudinal arm (132) extending along the barrel (100) and comprising an extremity provided with the triggering portion (134).

## Patentansprüche

1. Injektionsvorrichtung zum Injizieren einer Substanz in einen Körper, umfassend:
einen Zylinder (100), der zur Aufnahme der Substanz geeignet und mit einem Stopfen (103) in gleitendem Eingriff innerhalb des Zylinders (100) und mit einer Injektionsnadel (101) versehen ist;
eine Kolbenstange (110), die so konfiguriert ist, dass sie auf den Stopfen (103) drückt und bewegbar ist, um die Substanz zu injizieren, wenn eine Druckkraft auf die Kolbenstange (110) ausgeübt wird,
eine Verriegelungsklammer (150), die relativ zum Zylinder (100) befestigt ist und mit mindestens zwei Klemmbacken (151) versehen ist, die geeignet sind, die Kolbenstange (110) in einer Primärposition der Kolbenstange (110) in Eingriff zu nehmen, um eine Injektionsbewegung der Kolbenstange (110) in Bezug auf den Zylinder (100) zu verhindern, und
einen Nadelschutz (130), der einen Auslöseabschnitt (134) umfasst, wobei der Nadelschutz (130) in gleitendem Eingriff in Bezug auf die Injektionsnadel (101) konfiguriert ist, um eine Ausgangsposition, in der der Nadelschutz (130) die Injektionsnadel (101) abdeckt, und eine zurückgezogene Position, in der der Nadelschutz (130) zumindest einen Teil der Injektionsnadel (101) nicht abdeckt, zu definieren;
wobei der Auslöseabschnitt (134) so konfiguriert ist, dass er die Verriegelungsklammer (150) in Eingriff nimmt und diese öffnet, wenn sich der Nadelschutz (130) von der Ausgangsposition in die zurückgezogene Position bewegt, um die Verriegelungsklammer (150) von der Kolbenstange (110) zu lösen und die Injektionsbewegung der Kolbenstange (110) zu ermöglichen.

2. Injektionsvorrichtung nach dem vorstehenden Anspruch, wobei die Kolbenstange (110) einen Zwischenanschlag (114) aufweist und die beiden Klemmbacken (151) der Verriegelungsklammer (150) so angeordnet sind, dass sie in der Primärposition der Kolbenstange (110) den Zwischenanschlag (114) in Eingriff nehmen.

3. Injektionsvorrichtung nach einem der vorstehenden Ansprüche, wobei
der Nadelschutz (130) ferner in eine Sicherheitsposition bewegbar ist, in der der Nadelschutz (130) die Injektionsnadel (101) dauerhaft abdeckt, und
die Injektionsvorrichtung außerdem Folgendes umfasst:
einen Bedienungsanschlag (126a), der so konfiguriert ist, dass er verhindert, dass sich der Nadelschutz (130) in die Sicherheitsposition bewegt,
einen flexiblen Träger (126), der den Bedienungsanschlag (126a) trägt und in Bezug auf den Zylinder (100) befestigt ist,
ein Ablenkelement (160), das so konfiguriert ist, dass es den Bedienungsanschlag (126a) und/oder den flexiblen Träger (126) berührt und die Druckkraft von der Kolbenstange (110) aufnimmt, wenn die Kolbenstange (110) in eine Schwellenposition bewegt wird;
wobei das Ablenkelement (160) ferner so konfiguriert ist, dass es sich unter der Druckkraft bewegt, um den flexiblen Träger (126) abzulenken und den Bedienungsanschlag (126a) freizugeben.

4. Injektionsvorrichtung nach dem vorstehenden Anspruch, wobei das Ablenkelement (160) Folgendes umfasst:
eine Anschlagfläche (161), die so konfiguriert ist, dass sie die Druckkraft der Kolbenstange (110) aufnimmt, wenn sich die Kolbenstange in der Schwellenposition (110) befindet, und
einen abgeschrägten Abschnitt (164), der so konfiguriert ist, dass er auf dem flexiblen Träger (126) und/oder dem Bedienungsanschlag (126a) gleitet, um den flexiblen Träger (126) abzulenken, wenn sich das Ablenkelement (160) unter der Druckkraft der Kolbenstange (110) bewegt.

5. Injektionsvorrichtung nach dem vorstehenden Anspruch, wobei die Kolbenstange (110) eine Druckfläche (112b) aufweist, die geeignet ist, die Druckkraft auf die Anschlagfläche (161) des Ablenkelements (160) zu übertragen.

6. Injektionsvorrichtung nach einem der Ansprüche 3 bis 5, wobei die Injektionsvorrichtung ferner ein Gehäuse (120) umfasst, in dem die Verriegelungsklammer (150), der Zylinder (100), der Nadelschutz (130) und das Ablenkelement (160) untergebracht sind, und wobei der Bedienungsanschlag (126a) und der flexible Träger (126) an dem Gehäuse (120) befestigt sind oder Teil davon sind.

7. Injektionsvorrichtung nach dem vorstehenden Anspruch, wobei das Gehäuse (120) Folgendes umfasst:
eine distale Einheit (121), die den Nadelschutz (130) und den Zylinder (100) aufnimmt, und eine proximale Einheit (122), die die Verriegelungsklammer (150) und das Ablenkelement (160) aufnimmt.

8. Injektionsvorrichtung nach dem vorstehenden Anspruch, wobei die distale Einheit (121) des Gehäuses (120) ein Längsfenster (128) aufweist, das einen visuellen Zugang zum Zylinder (100) ermöglicht.

9. Injektionsvorrichtung nach einem der vorstehenden Ansprüche, wobei die Injektionsvorrichtung ferner elastische Mittel umfasst, die in schiebendem Eingriff mit dem Nadelschutz (130) angeordnet sind, um den Nadelschutz (130) aus der zurückgezogenen Position in die Ausgangsposition und/oder in die Sicherheitsposition zu bewegen.

10. Injektionsvorrichtung nach dem vorstehenden Anspruch, wenn dieser von den Ansprüchen 7 bis 9 abhängt, wobei die elastischen Mittel in der proximalen Einheit (122) des Gehäuses (120) untergebracht sind.

11. Injektionsvorrichtung nach Anspruch 6 oder nach einem der Ansprüche 7 bis 10, wenn diese von Anspruch 6 abhängen, wobei das Ablenkelement (160) eine Längslasche (166) aufweist, die so beschaffen ist, dass sie aus dem Gehäuse (120) herausspringt, wenn sich das Ablenkelement (160) unter der Druckkraft der Kolbenstange (110) bewegt.

12. Injektionsvorrichtung nach einem der Ansprüche 6 bis 11, ferner mit Sicherheitsverriegelungsmitteln, die geeignet sind, den Nadelschutz (130) am Gehäuse (120) zu verriegeln, wenn sich der Nadelschutz (130) in der Sicherheitsposition befindet.

13. Injektionsvorrichtung nach dem vorstehenden Anspruch, wobei die Sicherheitsverriegelungsmittel mindestens einen Zapfen (135), der an einem von dem Gehäuse (120) und dem Nadelschutz (130) vorgesehen ist, und mindestens eine Aussparung, die an dem anderen von dem Gehäuse (120) und dem Nadelschutz (130) vorgesehen ist, umfassen, wobei die Aussparung dazu geeignet ist, den Zapfen in der Sicherheitsposition des Nadelschutzes (130) aufzunehmen und zu halten.

14. Injektionsvorrichtung nach einem der vorstehenden Ansprüche, wobei der Nadelschutz (130) außerdem mindestens einen Längsarm (132) umfasst, der sich entlang des Zylinders (100) erstreckt und ein Ende aufweist, das mit dem Auslöseabschnitt (134) versehen ist.

## Revendications

1. Dispositif d'injection pour injecter une substance dans un corps, comprenant :
un corps cylindrique (100) adapté pour recevoir la substance et prévu avec un bouchon de piston (103) pour la mise en prise coulissante à l'intérieur du corps cylindrique (100) et avec une aiguille d'injection (101);
une tige de piston (110) configurée pour pousser le bouchon de piston (103) et mobile afin d'injecter la substance lorsqu'une force de poussée est appliquée sur la tige de piston plongeur (110),
une attache de verrouillage (150) fixe par rapport au corps cylindrique (100) et prévue avec au moins deux mâchoires (151) adaptées pour mettre en prise la tige de piston (110) dans une position principale de la tige de piston (110), afin d'empêcher un mouvement d'injection de la tige de piston (110) par rapport au corps cylindrique (100), et
une protection d'aiguille (130) comprenant une partie de déclenchement (134), ladite protection d'aiguille (130) étant configurée pour la mise en prise coulissante par rapport à l'aiguille d'injection (101), afin de définir une position initiale, dans laquelle la protection d'aiguille (130) recouvre l'aiguille d'injection (101) et une position rétractée, dans laquelle la protection d'aiguille (130) ne recouvre pas au moins une partie de l'aiguille d'injection (101) ;
dans lequel la partie de déclenchement (134) est configurée pour mettre en prise et ouvrir l'attache de verrouillage (150) lorsque la protection d'aiguille (130) passe de la position initiale à la position rétractée, afin de dégager l'attache de verrouillage (150) de la tige de piston (110) et afin de permettre le mouvement d'injection de la tige de piston (110).

2. Dispositif d'injection selon la revendication précédente,
dans lequel la tige de piston (110) comprend une butée intermédiaire (114) et les deux mâchoires (151) de l'attache de verrouillage (150) sont agencées pour mettre en prise ladite butée intermédiaire (114) dans la position principale de la tige de piston (110).

3. Dispositif d'injection selon l'une quelconque des revendications précédentes, dans lequel :
la protection d'aiguille (130) est en outre mobile dans une position de sécurité dans laquelle la protection d'aiguille (130) recouvre, de manière permanente, l'aiguille d'injection (101), et
le dispositif d'injection comprend en outre :
une butée opérationnelle (126a) configurée pour empêcher la protection d'aiguille (130) de se déplacer dans la position de sécurité,
un support flexible (126) supportant la butée opérationnelle (126a) et fixe par rapport au corps cylindrique (100),
un élément de déviation (160) configuré pour entrer en contact avec la butée opérationnelle (126a) et/ou le support flexible (126) et pour recevoir la force de poussée de la tige de piston (110), lorsque la tige de piston (110) est déplacée dans une position de seuil ;
dans lequel l'élément de déviation (160) est en outre configuré pour se déplacer sous ladite force de poussée afin de dévier le support flexible (126) et de libérer la butée opérationnelle (126a).

4. Dispositif d'injection selon la revendication précédente, dans lequel l'élément de déviation (160) comprend :
une surface de butée (161) configurée pour recevoir la force de poussée de la tige de piston (110) lorsque la tige de piston plongeur est dans la position de seuil (110), et
une partie inclinée (164) configurée pour coulisser sur le support flexible (126) et/ou la butée opérationnelle (126a) afin de dévier le support flexible (126) lorsque l'élément de déviation (160) se déplace sous la force de poussée de la tige de piston (110).

5. Dispositif d'injection selon la revendication précédente, dans lequel la tige de piston (110) comprend une surface de poussée (112b) adaptée pour transmettre la force de poussée à la surface de butée (161) de l'élément de déviation (160).

6. Dispositif d'injection selon l'une quelconque des revendications 3 à 5, dans lequel le dispositif d'injection comprend en outre un boîtier (120) logeant l'attache de verrouillage (150), le corps cylindrique (100), la protection d'aiguille (130) et l'élément de déviation (160), et dans lequel la butée opérationnelle (126a) et le support flexible (126) sont fixés sur ou font partie du boîtier (120).

7. Dispositif d'injection selon la revendication précédente, dans lequel le boîtier (120) comprend :
une unité distale (121) logeant la protection d'aiguille (130) et le corps cylindrique (100) et une unité proximale (122) logeant l'attache de verrouillage (150) et l'élément de déviation (160).

8. Dispositif d'injection selon la revendication précédente, dans lequel l'unité distale (121) du boîtier (120) comprend une fenêtre longitudinale (128) donnant un accès visuel sur le corps cylindrique (100).

9. Dispositif d'injection selon l'une quelconque des revendications précédentes, dans lequel le dispositif d'injection comprend en outre des moyens élastiques agencés en mise en prise par poussée avec la protection d'aiguille (130) afin de déplacer la protection d'aiguille (130) de la position rétractée à la position initiale et/ou à la position de sécurité.

10. Dispositif d'injection selon la revendication précédente lorsqu'elle dépend des revendications 7 à 9, dans lequel les moyens élastiques sont logés dans l'unité proximale (122) du boîtier (120).

11. Dispositif d'injection selon la revendication 6 ou l'une quelconque des revendications 7 à 10 lorsqu'elles dépendent de la revendication 6, dans lequel l'élément de déviation (160) comprend une languette longitudinale (166) adaptée pour surgir du boîtier (120), lorsque l'élément de déviation (160) se déplace sous la force de poussée de la tige de piston (110).

12. Dispositif d'injection selon l'une quelconque des revendications 6 à 11, comprenant en outre des moyens de verrouillage de sécurité adaptés pour verrouiller la protection d'aiguille (130) sur le boîtier (120), lorsque la protection d'aiguille (130) est dans la position de sécurité.

13. Dispositif d'injection selon la revendication précédente, dans lequel les moyens de verrouillage de sécurité comprennent au moins une cheville (135) prévue sur l'un parmi le boîtier (120) et la protection d'aiguille (130) et au moins un évidement prévu sur l'autre parmi le boîtier (120) et la protection d'aiguille (130), l'évidement étant adapté pour loger et maintenir la cheville dans la position de sécurité de la protection d'aiguille (130).

14. Dispositif d'injection selon l'une quelconque des revendications précédentes, dans lequel la protection d'aiguille (130) comprend en outre au moins un bras longitudinal (132) s'étendant le long du corps cylindrique (100) et comprenant une extrémité prévue avec la partie de déclenchement (134).
